# EUROPEAN PATENT APPLICATION

(11) **EP 4 029 853 A1**
(43) Date of publication of application: **20.07.2022**
(21) Application number: 20863253.9
(22) Date of filing: 10.09.2020
(51) Int. Cl.: C07D 213/89, C07C 209/58, C07C 213/06, C07D 213/127, C07D 213/12, C07D 213/04, C07C 255/07, A61P 35/00, A61K 31/33

(54) **SYNTHESIS METHOD APPLIED TO KRAS INHIBITOR DRUG HETEROCYCLIC INTERMEDIATE**

(30) Priority: 11.09.2019 CN 201910858876
(71) Applicant: Chiral Quest (suzhou) Co., Ltd., Suzhou Industrial Park Suzhou Jiangsu 215123 (CN); Jiangxi Longlife Bio-Pharmaceutical Co., Ltd., Jinxian Industrial Park Nanchang Jiangxi 331700 (CN)
(72) Inventor: WU, Shengwen, Suzhou, Jiangsu 215123 (CN); LI, Wenge, Suzhou, Jiangsu 215123 (CN); ZHOU, Peng, Suzhou, Jiangsu 215123 (CN)
(74) Representative: karo IP
(86) International application number: PCT/CN2020/114560
(87) International publication number: WO 2021/047603

(57) **Abstract**

The present application provides a compound of formula II and a method for preparing a compound of formula I, i.e., 2-iso-propyl-4-methylpyridin-3-amine by using the same. The compound of formula I can be applied to synthesis of KRAS inhibitor drugs. The raw materials involved in the method of the present application are easy to obtain and low in price, operation is easy and convenient, economization and environmental protection are achieved, and industrial production is facilitated.

## Description

### RELATED APPLICATIONS

This application claims the priority of Chinese patent application 2019108588762 filed on September 11th, 2019. This application refers to the full text of the above Chinese patent application

### TECHNICAL FIELD OF THE INVENTION

This application is related to the pharmaceutical synthesis field, in particular, a synthetic method applied to heterocyclic intermediates of KRAS inhibitor drugs.

### BACKGROUND OF THE INVENTION

KRAS mutation is very common in human cancers (around 30%). It is one of the most common tumor driven genes, and has a high incidence in lung, colon and pancreatic cancers. However, due to the lack of drug binding pocket of KRAS mutant protein, it is difficult to design a KRAS inhibitor drug.

Amgen announced the phase I clinical results of small molecule KRAS inhibitor AMG510 at 2019 ASCO annual meeting, which achieved a historic breakthrough. Then the importance of 2-isopropyl-3-amino-4-methylpyridine (compound I) as an indispensable fragment of AMG510 has also been highlighted.

So far, there are few related patents reported. The main synthetic route in patents is the synthetic route of the innovator, Amgen (WO2018217651):

In the example 40 of WO2018217651 (page 235), high catalyst loading and the expensive Palladium catalyst and Zinc reagent are used in the coupling reaction. Meanwhile, the compound XI is not commercially available. It's synthesis is reported in Journal fuer Praktische Chemie (Leipzig) paper (331 (1989), 369-374) with 46% yield.

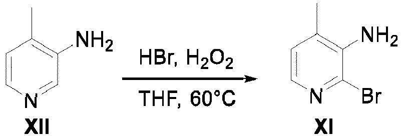

In order to improve the synthesis of compound I, the synthetic route needs to be re-designed to change the expensive transition-metal catalyzed cross-coupling reaction, in particular, avoid the amino group in the substrate which poisons the activity of the cross-coupling catalysts. Furthermore, if the cross-coupling reaction could be eliminated, the raw material cost of this pharmaceutical intermediate would be effectively reduced.

### SUMMARY OF THE INVENTION

The first objective of this application is to provide compounds of formula II, V and VI, preparation methods thereof, and the application to the intermediate of KRAS inhibitor.

Another objective of this application is to provide the preparation method for compounds of formula I.

The present application provides the following technical schemes to solve the problems.

In one aspect, the present application provides compounds of formula II, V 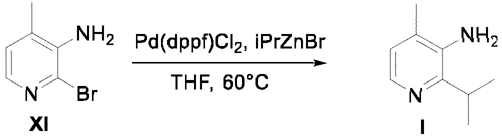 and VI:

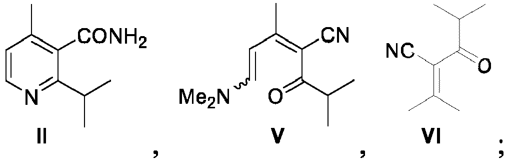

In formula V, the double bond of dimethylamine group are Z-configuration, E-configuration, or the mixture of Z-and E-configuration.

The present application provides compounds as intermediates of KRAS inhibitors,structures of which are shown in formula II, V and VI:

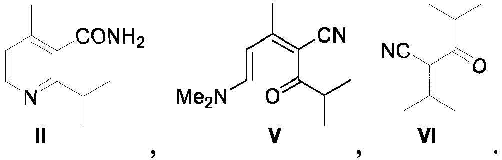

In another aspect, the present application provides methods for synthesis compound of formula I from compound of formula II, comprising the decarboxylation of compound of formula II via rearrangement reaction:

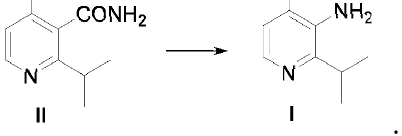

In one scheme in this application, the synthesis of compound of formula I comprises the following step:
Compound II reacts under the Hoffman rearrangement reaction conditions to produce compound I. The Hoffman rearrangement reaction conditions include a mixed system of NaOBr and water.

Wherein, NaOBr can be used in form of aqueous solution of NaOBr, which is prepared by adding Br₂ to the mixture of NaOH and water. The mass ratio of NaOH to Br₂ is 0.9:1, the mass ratio of NaOH to water in the mixture of NaOH and water is 0.3:1.

The molar ratio of compound II to NaOBr is (0.8-1.2):1, for example (0.9-1.1):1. When NaOBr aqueous solution prepared in the above steps is used, the mass ratio of compound II to Br₂ is 1:1

The mixed system can be obtained by the following step: adding NaOBr to the mixture of compound II and water. The addition method is dropping. The addition temperature is -10 to 10 °C, such as 0 °C. In the mixture of compound II and water, the mass ratio of compound II to water is 0.5:1.

In the mixed system, the mass ratio of the total amount of water (including water in the NaOBr aqueous solution, when NaOBr aqueous solution is used) to the compound II is 7:1.

The reaction temperature of the rearrangement reaction is 10 to 100 °C, for example room temperature (10-30 °C) to 80 °C.

The progress of the rearrangement reaction can be monitored by the conventional test methods in the art (for example, TLC, HPLC or NMR), and the reaction reaches the end-point when compound II disappears or no more reaction occurs. The reaction time is 1-5 hours.

The work-up is included in the synthetic method, which comprises following steps: after the rearrangement reaction is completed, the reaction mixture is extracted with organic solvent. Then the obtained organic phase is rinsed, concentrated, isolated and purified. The organic solvent for extraction is ethyl acetate. The rinsing solution is saturated brine. The isolation and purification method is silica gel column chromatography, for example, column chromatography using ethyl acetate and n-heptane (volume ratio 1:2) as eluent.

In one solution in this application, the synthesis of compound of formula II comprises the following step, hydrolysis of Compound III in acid to obtain compound II.

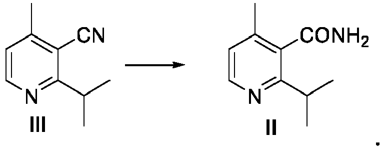

The conditions and operations of the hydrolysis reaction can be conventional conditions and operations in such reaction in the art; In the present application, the following are preferred:
The acid is concentrated sulfuric acid, for example 98% concentrated sulfuric acid.

The mass ratio of the acid to the compound III is 2.25:1.

The hydrolysis temperature is 105 °C.

The progress of the hydrolysis reaction can be monitored by the conventional test methods in the art (for example, TLC, HPLC or NMR), and the reaction reaches the end-point when compound III disappears or no more reaction occurs. The reaction time is 1-5 hours.

The work-up is included in the synthetic method, which comprises following steps: after hydrolysis reaction is completed, add water and adjust pH to 10-11, filter and dry to obtain compound II. 50% sodium hydroxide aqueous solution is used to adjust pH. The product of compound II can be directly used in the rearrangement reaction.

In one solution in this application, the synthesis methods comprise the scheme 1 and 2.

The scheme 1 includes the following step, in the presence of N-methyl-pyrrolidone and iron catalyst, the Kumada coupling reaction of compound IV with isopropyl Grignard reagent in an organic solvent, to produce compound III.

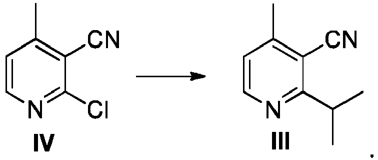

The conditions and operations of the Kumada coupling reaction can be conventional conditions and operations in such reaction in the art; In the present application, the following are preferred:
The organic solvent is ether solvents, such as tetrahydrofuran

It is not necessary to set limit for the amount of the organic solvents, as long as it does not affect the reaction. For example, the mass-to-volume ratio of compound IV to the organic solvents is 0.03-0.04 g/mL.

The mass ratio of N-methyl-pyrrolidone to compound IV is 9.5:1

The iron catalyst is Iron (III) acetylacetonate. The mass ratio of the iron catalyst to compound IV is 1:(2-2.2)

The isopropyl Grignard reagent is isopropyl magnesium chloride. The isopropyl Grignard reagent can be used in solution form. The solvent of the solution is organic solvents, such as ether solvent. For example 1.0-2.5 mol/L tetrahydrofuran solution.

The volume-to-mass ratio of the isopropyl Grignard reagent to compound IV is (2.6-4.4):1.

The reaction temperature of the Kumada coupling is 0-10 °C.

The progress of the Kumada coupling can be monitored by the conventional test methods in the art (for example, TLC, HPLC or NMR), and the reaction reaches the end-point when compound IV disappears or no more reaction occurs. The reaction time can be 0.2-5 hours.

The work-up is included in the synthetic method, which comprises following steps: after Kumada coupling reaction is completed, add citric acid aqueous solution and saturated sodium bicarbonate aqueous solution to the reaction mixture successfully, extract with organic solvents. Then the organic phase is washed, concentrated, isolated and purified. The organic solvent for extraction is ester solvents, such as ethyl acetate. The isolation and purification method is silica gel column chromatography, for example column chromatography using ethyl acetate and n-heptane (volume ratio 1:6) as eluent. The product of compound III can be directly used in the hydrolysis reaction.

The scheme 2 includes the following step, the compound V is cyclized with ammonia source in organic solvent to obtain the compound III.

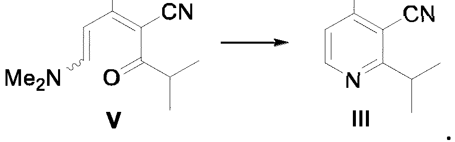

In scheme 2, the cyclization reaction is the reaction of compound V with ammonia source, followed by intramolecular substitution reaction to produce compound III.

The conditions and operations of the cyclization reaction can be conventional conditions and operations in such reaction in the art; In the present application, the following are preferred:
The organic solvent is alcohol solvents, such as methanol.

It is not necessary to set limit for the amount of the organic solvents, as long as it does not affect the reaction. For example, the mass-to-volume ratio of compound V to the organic solvent is 0.03-0.04 g/mL.

The ammonia sources can be ammonia, ammonium acetate, ammonium chloride; for example, when the ammonia source is ammonium acetate, the mass ratio of the ammonium acetate to compound V is (3-4):1, for example 3.67:1.

The reaction temperature of cyclization reaction is 0-50 °C, for example 20-30 °C.

The progress of cyclization reaction can be monitored by the conventional test methods in the art (for example, TLC, HPLC or NMR), and the reaction reaches the end-point when compound V disappears or no more reaction occurs. The reaction time is 1-10 days, for example 5 days.

The workup is included in the synthetic method, which comprises following steps: after cyclization reaction is completed, the reaction mixture is concentrated, isolated and purified to obtain product of compound III. The isolation and purification method is silica gel column chromatography, for example column chromatography using ethyl acetate and n-heptane (volume ratio 1:6) as eluent. The product of compound III can be directly used in the hydrolysis reaction.

In another solution of this application, the scheme 2 includes the following steps.
Step (1), in organic solvent, compound VII is condensed with acetone in the presence of alkaline aluminum oxide to obtain compound VI;
Step (2), in organic solvent, compound VI is condensed with N,N-dimethylformamide dimethyl sulfate condensate (DMF-DMS) in the presence of acetic anhydride and triethylamine to obtain compound V.

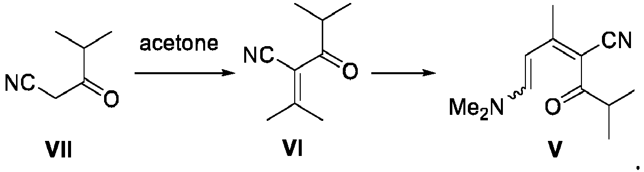

The conditions and operations of the condensation reaction of steps (1) and (2) can be conventional conditions and operations in such reaction in the art; In the present application, the following are preferred:
The organic solvent used in step (1) is aromatic solvents, such as toluene.

It is not necessary to set limit for the amount of the organic solvents in step (1), as long as it does not affect the reaction. For example, the mass-to-volume ratio of compound VII to the organic solvent can be 0.10-0.20 g/mL.

In step (1), the mass ratio of the alkaline aluminum oxide to compound VII is (2-4):1, for example 2.3:1.

The reaction temperature of condensation reaction in step (1) is 0-50 °C, for example 20-30 °C.

In step (1), the progress of condensation reaction can be monitored by the conventional test methods in the art (for example, TLC, HPLC or NMR), and the reaction reaches the end-point when compound VII disappears or no more reaction occurs. The reaction time is 0.5-2 days, for example 1 day.

The workup of step (1) is included in the synthetic method, which comprises following steps: after condensation reaction is completed, filter the reaction mixture, rinse the filter cake with organic solvents, combine the filtrate and directly use it in step (2).

The organic solvent used in step (2) is aromatic solvents, such as toluene.

In step (2), the mass ratio of N,N-dimethylformamide dimethyl sulfate condensate (DMF-DMS) to compound VII is (2-4):1, for example 3:1.

In step (2), the mass ratio of acetic anhydride to compound VII is (0.1-0.4):1, for example 0.18:1.

In step (2), the volume-to-mass ratio of triethyl amine to compound VII is 1-2 mL/g, for example 1.5 mL/g.

The reaction temperature of condensation reaction in step (2) is 0-50 °C, for example 0-10 °C to 20-30 °C.

In one scheme, step (2) comprises be the following steps: to the mixture of compound VI and N,N-dimethylformamide dimethyl sulfate condensate obtained in step (1), acetic anhydride and triethylamine are successively added for condensation reaction; The addition temperature is 0-10 °C for acetic anhydride, and 20-30 °C for triethylamine.

The progress of condensation reaction in step (2) can be monitored by the conventional test methods in the art (for example, TLC, HPLC or NMR), and the reaction reaches the end-point when compound VI disappears or no more reaction occurs. The reaction time is 5-24 hours, for example 20 hours.

The work-up is included in the procedure of step (2), which comprises following steps: after condensation reaction is completed, add water to the reaction mixture, stir for 5 h, separate the layers, extract the water phase with organic solvent, combine organic phase, condense, and purify to obtain product of compound V. The organic solvent for extraction is dichloromethane. The isolation and purification method is silica gel column chromatography, for example column chromatography using ethyl acetate and n-heptane (volume ratio 1:6) as eluent. The product of compound V can be directly used in cyclization reaction.

In the other aspect, the application provides a synthetic method of compound II including coupling reaction of compound IV to produce compound III, then hydrolysis of compound III. The method comprises the following steps:

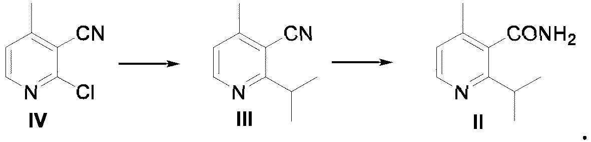

The conditions and operations of the coupling and hydrolysis reactions can be the conditions and operations of the corresponding reactions described above

In a specific coupling reaction embodiment for the synthesis of compound III, the iron catalyzed is used in the Kumada coupling, and compound IV and isopropyl Grignard reagent are reactants.

In another scheme, the application provides a synthetic method for compound II. The method comprises the following steps, condensation reaction of compound VII with acetone to make compound VI, producing compound V from compound VI by condensation reaction, then cyclization reaction of compound V to lead compound III, finally hydrolysis reaction of compound III to produce compound II.

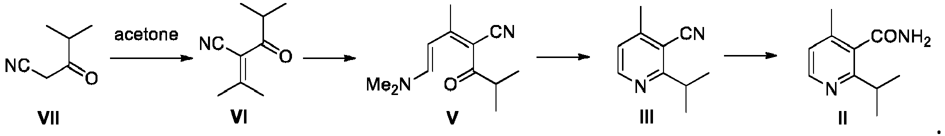

The conditions and operations of the condensation, cyclization and hydrolysis reactions can be the conditions and operations of corresponding reactions described above.

In a specific embodiment of the synthesis of compound V through condensation of compound VI, compound VI and N,N-Dimethylformamide dimethyl acetal (DMF-DMA) or N,N-dimethylformamide dimethyl sulfate condensate (DMF-DMS) are used as reactants of condensation reaction. And in the synthesis of compound III through compound V, cyclization reaction includes the reaction of compound V with ammonia source, followed by intramolecular substitution reaction to produce compound III. More preferably, the ammonia source is selected from ammonia, ammonium acetate and ammonium chloride.

Compared with the prior art, the synthetic method of compound of formula I in the present application has the following advantages:
1. Raw materials are easily available and cheap.
2. The operation is simple and the process is easy for scale-up and industrial production.
3. Safer and more environmentally friendly.

### DETAILED DESCRIPTION OF EMBODIMENTS

The following embodiments describe the implementation of the present application, and those skilled in the art should realize that these specific embodiments only show the implementation technical solutions selected to achieve the purpose of this application and are not limitations on the technical solutions. According to the teaching of this application, the improvement of the technical solution of this application in combination with the prior art is obvious, which all fall within the protection scope of this application.

The implementation conditions employed by the embodiments may be further adjusted according to particular requirements, and undefined implementation conditions usually are conditions in conventional experiments.

Among them, the known chemical reagents used in the following embodiments are all commercially available chemical reagents.

In the exemplary implementations of the present disclosure, those skilled in the art can also make changes to the synthetic route, for example, change specific reaction conditions or adjust a certain step or several steps of the synthetic route as needed. Any changes made without departing from the substance of this application are within the protection scope of this application.

### Example 1

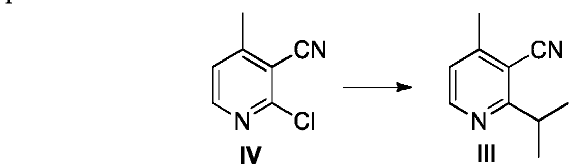

To a 500 mL three-necked flask were added compound IV (6.0 g), THF (150 mL), NMP (57 mL) and Fe(acac)₃ (2.77 g). The reaction mixture was refilled with nitrogen for three times, cooled to 0~ 10 °C. ⁱPrMgCl in THF (39 mL) was added to the reaction mixture dropwise at 0~10°C. After being stirred for 30mins, the reaction was quenched with citric acid aqueous solution (60 mL, 0.5 mol/L), followed by addition of saturated NaHCO₃ aqueous solution (60 mL) and EtOAc (60 mL). The layers were separated. The aqueous layer was extracted with EtOAc (60 mL). The organic phases were combined and concentrated under reduced pressure. Compound 3 (3.59 g) was obtained as an oily liquid with a yield of 57% via column chromatography purification (EtOAc: n-heptane=1:6).

¹H NMR for compound III:
¹H NMR (400 MHz, DMSO-d₆) δ 8.63 (d, J = 5.1 Hz, 1H), 7.35 (dd, J = 5.0, 0.8 Hz, 1H), 3.41 (m, J = 6.8 Hz, 1H), 2.50 (s, J = 0.6 Hz, 3H), 1.27 (d, J = 6.8 Hz, 6H).

MS for compound III: [M+H]⁺=161.1.

### Example 2

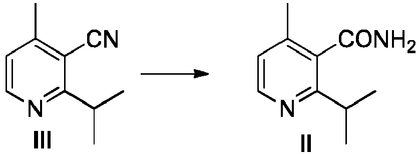

To compound III (3.0 g) in a 50 mL reaction flask, concentrated sulfuric acid (6.76 g) was added dropwise. The mixture was heated to 105 °C and stired for 4 h. After the reaction was completed, the reaction was cooled to 70~75 °C. Water (6 g) was added to the reaction. After 30 minutes, the reaction was cooled down to 0~10 °C. Water (21 g) and 50% sodium hydroxide aqueous solution were added to adjust the reaction mixture pH =10~11. After 30 min, the mixture was filtered. The cake was dried at 50 °C to obtain off-white solid (2.5 g, 75% yield).

¹H NMR for compound II:
¹H NMR (400 MHz, DMSO-d₆) δ 8.38 (d, J = 4.9 Hz, 1H), 7.90 (s, 1H), 7.63 (s, 1H), 7.08 (d, J = 4.7 Hz, 1H), 3.14 (m, 1H), 2.26 (s, 3H), 1.20 (d, J = 6.8 Hz, 6H).

MS for compound II: [M+H]⁺=179.1.

### Example 3:

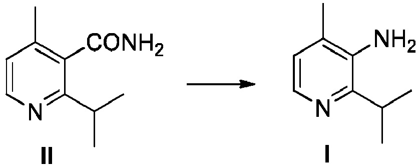

To a mixture of NaOH (0.45 g) and water (1.5 mL) in a 25 mL round-bottom flask A, Br₂ (0.5 g) was added dropwise in an ice-water bath. After being stirred for 10 min at room temperature, the resulting NaOBr aqueous solution was added to a mixutre of compound II (0.5 g) and water (1 mL) in a 25 mL round-bottom flask B in an ice-water bath. After being stired for 1.5h at room temperature, water (1 mL) was added. The reaction mixture was heated to 80 °C and stired for 1.5 h. After the reaction completed, the reaction was cooled to 20~30 °C, EtOAc (10 mL) was added. The layers were separated. The aqueous phase was extracted with EtOAc (10 mL). The combined organic phases were washed with saturated brine (10 mL) and concentrated. The crude product was purified by column chromatography (EtOAc: n-heptane=1:2) to afford compound I(140 mg, 33% yield) as a pale yellow oily liquid.

¹H NMR for compound I:
¹H NMR (400 MHz, DMSO-d₆) δ 7.67 (d, J = 4.7 Hz, 1H), 6.78 (d, J = 4.6, 0.8 Hz, 1H), 4.72 (s, 2H), 3.19 (m, 1H), 2.08 (s, 3H), 1.15 (d, J = 6.7 Hz, 6H).

MS for compound I: [M+H]⁺=151.2.

### Example 4:

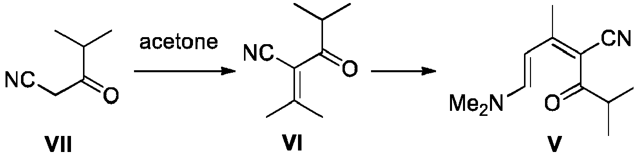

To a mixture of compound VII (5.0 g) and acetone (6.7 mL) in a 100 mL round-bottom flask A, basic Al₂O₃ (11.5 g) and toluene (30 mL) were added. The reaction was stirred at 20-30 °C for 24 hours. After the reaction was completed, the mixture was filtered. The cake was rinsed with toluene (20 mL), the filtrate was transferred to 250 mL round-bottom flask B. N,N-dimethylformamide dimethyl sulfate adduct (DMF-DMS) (15.4 g), acetic anhydride (0.92 g) was added. The mixture was cooled to 0-10 °C, followed by addition of triethylamine (7.5 mL) dropwise. The reaction was stirred at 20-30 °C for 15 h. Water (40 mL) was added. The mixture was stirred for another 5 h. The layers were separated. The aqueous phase was extracted with DCM (3 x 30 mL). The combined organic phases were concentrated. The crude material was purified by column chromatography (EtOAc: n-heptane = 1: 10) to afford compound V (3.0 g, 32%) as a light yellow solid.

¹H NMR for compound V:
¹H NMR (400 MHz, DMSO-d₆) δ 7.96 (d, J = 12.8 Hz, 1H), 7.55 (d, J = 12.8 Hz, 1H), 3.31 (d, J = 2.2 Hz, 1H), 3.25 (s, 3H), 3.13 (m, 1H), 2.97 (s, 3H), 2.24 (s, 3H), 1.01 (dd, J = 6.8, 3.6 Hz, 7H).

MS for compound V: [M+H]⁺=207.1.

### Example 5:

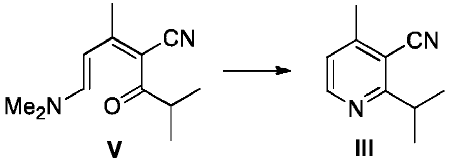

A mixture of compound V (60 mg), methanol (1.8 mL), and ammonium acetate (0.22 g) in a 10 mL round-bottom flask was stirred at 20-30 °C for 5 days. After the reaction was completed, the reaction mixture was concentrated under reduced pressure. The crude product was purified by column chromatography (EtOAc: N-heptane=1:6) to afford compound III (23 mg, 50% yield) as an oily liquid.

¹H NMR for compound III:
¹H NMR (400 MHz, DMSO-d₆) δ 8.63 (d, J = 5.1 Hz, 1H), 7.35 (dd, J = 5.0, 0.8 Hz, 1H), 3.41 (m, 1H), 2.50 (s, J = 0.6 Hz, 3H), 1.27 (d, J = 6.8 Hz, 6H).

MS for compound III: [M+H]⁺=161.1.

This application includes but is not limited to the above embodiments. Any equivalent substitution or partial improvement made under the principle of the spirit of this application will be deemed to be within the protection scope of this application.

## Claims

1. A compound having a formula II, V, or VI: 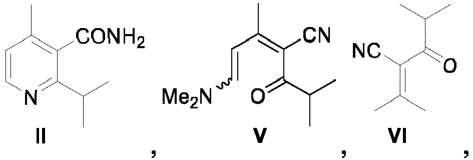 or a salt thereof wherein, in formula V, the double bond connected with the dimethylamino group is in a form of Z, E, or a mixture of Z and E.

2. A compound or salt thereof as a KRAS inhibitor drug intermediate, wherein the compound has a structure of formula II, V, or VI: 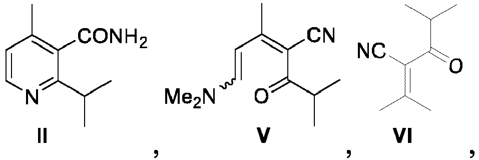 wherein, in formula V, the double bond connected with the dimethylamino group is in the form of Z, E, or a mixture of Z and E.

3. A synthetic method for preparing a compound of formula I from a compound of formula II, **characterized in that**, compound I is prepared by converting an amide group to amino group from compound II through a rearrangement reaction, and the method comprises the following step:

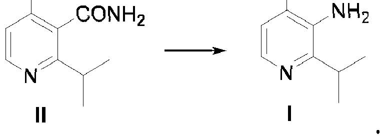

4. The synthetic method according to claim 3, **characterized in that** the method comprises the following steps:
reacting the compound II under the conditions of Hoffman rearrangement reaction to obtain the compound I; wherein the conditions of the Hoffman rearrangement reaction may be a mixed system of NaOBr and water.

5. The synthetic method according to claim 4, wherein the NaOBr is used in the form of an aqueous solution of NaOBr;
and/or, the NaOBr is prepared by the following steps: Br₂ is added to the mixture of NaOH and water to obtain the NaOBr aqueous solution; wherein the mass ratio of NaOH to Br₂ can be 0.9:1; in the mixture of NaOH and water, the mass ratio of NaOH to water can be 0.3:1; when NaOBr is the NaOBr aqueous solution prepared in the above steps, the mass ratio of the compound II to Br₂ can be 1: 1; and/or, the molar ratio of compound II to NaOBr is (0.8-1.2):1; for example (0.9-1.1):1;
and/or, the mixing system is obtained by the following steps: NaOBr is added to the mixture of compound II and water; the addition can be dripping; the temperature of the addition can be- 10°C to 10°C, such as 0°C; in the mixture of the compound II and water, the mass ratio of the compound II to the water can be 0.5:1;
and/or, in the mixed system, the mass ratio of the total amount of water to the compound II is 7:1;
and/or, the reaction temperature of the rearrangement reaction is 10°C to 100°C; for example, 10°C to 80°C;
and/or, the synthetic method further includes process of work-up, and the process of work-up comprises the following steps: after the rearrangement reaction is completed, an organic solvent is added for extraction, the obtained organic phase is washed and concentrated, separation and purification; the organic solvent in the extraction can be ethyl acetate; the washing solvent can be saturated brine; the separation and purification can be silica gel column chromatography; for example, ethyl acetate and n-heptane are used; the volume ratio of the eluent of ethyl acetate and n-heptane can be 1:2.

6. The synthetic method according to claim 3, **characterized in that**, the method comprises the following step: hydrolyzing the compound III with an acid to obtain the compound II:

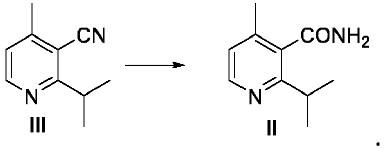

7. The synthetic method according to claim 6, wherein the acid is sulfuric acid;
and/or, the mass ratio of the acid to the compound III is 2.25:1;
and/or, the temperature of the hydrolysis reaction is 105°C;
and/or, in the synthetic method also includes a process of work-up, and the process of work-up comprises the following steps: after the hydrolysis reaction is completed, water is added to adjust the pH to 10-11; filtered and dried to obtain the compound II; the pH can be adjusted by adding 50% sodium hydroxide aqueous solution; the compound II can be directly used in the rearrangement reaction.

8. The synthetic method according to claim 6, **characterized in that** the method comprises the following scheme 1 or scheme 2;
Scheme 1 includes the following step, in the presence of N-methylpyrrolidone and iron catalyst, conducting Kumada coupling reaction of compound IV with isopropyl Grignard reagent in an organic solvent, to obtain the compound III. 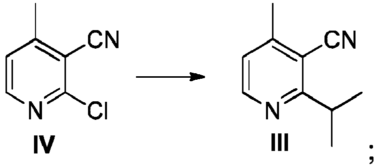
Scheme 2 includes the following steps in an organic solvent, subjecting the compound V and an ammonia source to a cyclization reaction to obtain the compound III;

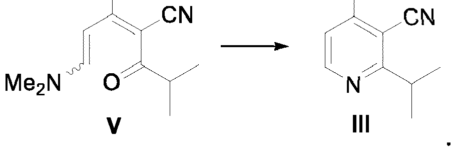

9. The synthetic method according to claim 8, **characterized in that**:
in scheme 2, wherein the cyclization reaction comprises reacting the compound V and an ammonia source followed by an intra-molecular substitution reaction to form the compound III;
and/or, in scheme 1, the organic solvent is an ether solvent, such as tetrahydrofuran;
and/or, in scheme 1, the mass-volume ratio of the compound IV to the organic solvent is 0.03-0.04 g/mL;
and/or, in scheme 1, the volume-to-mass ratio of said N-methylpyrrolidone to said compound IV is 9.5:1;
and/or, in scheme 1, the iron catalyst is iron triacetylacetonate;
and/or, in scheme 1, the mass ratio of the iron catalyst to the compound IV is 1:(2-2.2);
and/or, in scheme 1, the isopropyl Grignard reagent is isopropyl magnesium chloride;
and/or, in scheme 1, the isopropyl Grignard reagent is used in the form of a solution; the solvent of the solution can be the organic solvent, for example, an ether solvent; for example, 1.0~2.5 mol/ L tetrahydrofuran solution;
and/or, in scheme 1, the volume-to-mass ratio of said isopropyl Grignard reagent to said compound IV is (2.6-4.4):1;
and/or, in scheme 1, the temperature of the Kumada coupling reaction is 0~10°C;
and/or, in scheme 1, it also includes the process of work-up, and the process of work-up is the following steps: after the completion of the Kumada coupling reaction, add citric acid aqueous solution, saturated sodium bicarbonate aqueous solution, and organic solvent in sequence; extraction, concentration of the organic phase, separation and purification to obtain the compound III; the organic solvent for extraction can be an ester solvent, such as ethyl acetate; the separation and
purification can be silica gel column chromatography; for example, eluent ratio of ethyl ester and n-heptane can be 1:6.

10. The synthetic method according to claim 8 **characterized in that** the method comprises:
step (1): in an organic solvent, in the presence of basic alumina, subjecting compound VII to a condensation reaction with acetone to obtain compound VI;
step (2): in an organic solvent, in the presence of acetic anhydride and triethylamine, subjecting the compound VI to a condensation reaction with the N,N-dimethylformamide dimethyl sulfate condensate to obtain the compound V;

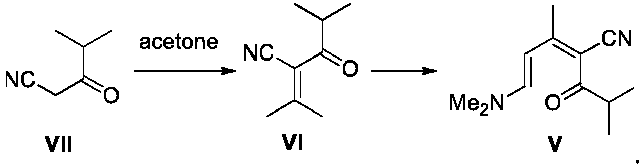

11. The synthetic method according to claim 10, wherein the organic solvent in step (1) is an aromatic solvent; for example, toluene;
and/or, in step (1), the mass-volume ratio of the compound VII to the organic solvent is 0.10-0.20 g/mL;
and/or, in step (1), the mass ratio of the basic alumina to the compound VII is (2-4):1; for example, 2.3:1;
and/or, in step (1), the temperature of the condensation reaction is 0-50°C; for example, 20-30°C;
snd/or, step (1) also includes s work-up process, wherein the work-up process comprises the following steps: when the condensation reaction is completed, filtering, washing the filter cake with the organic solvent, and combining the resulting filtrate directly used in step (2);
and/or, in step (2), the organic solvent is aromatic solvent; for example, toluene; and/or, in step (2), the mass ratio of the N,N-dimethylformamide dimethyl sulfate condensate to the compound VII is (2-4):1; for example, 3:1;
and/or, in step (2), the mass ratio of the acetic anhydride to the compound VII is (0.1-0.4):1; for example, 0.18:1;
and/or, in step (2), the mass-to- volume ratio of the triethylamine to the compound VII is 1-2 mL/g; for example, 1.5 mL/g;
and/or, in step (2), the temperature of the condensation reaction can be 0~50°C; for example, 0~10°C to 20~30°C;
and/or, step (2) is the following step: acetic anhydride and triethylamine are sequentially added to the compound VI obtained in step (1) to condense with N,N-dimethylformamide dimethyl sulfate In the mixture of substances, the condensation reaction is carried out; wherein the temperature of adding acetic anhydride can be 0~10°C; the temperature of adding triethylamine can be 20~30°C;
and/or, step (2) also includes a work-up process, wherein the work-up process comprises the following steps: when the condensation reaction is completed, water is added and stirred, the layers are separated, the aqueous phase is extracted with an organic solvent, and the organic phases are combined Concentrate, separate and purify to obtain the compound V; the organic solvent for extraction can be dichloromethane; the separation and purification can be silica gel column chromatography; for example, elution with ethyl acetate and n-heptane Liquid, the volume ratio can be 1:10.

12. A method for preparing a compound of formula II comprising a hydrolysis reaction of compound III: 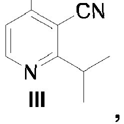 wherein, the reaction conditions and process are as described in any one of claims 6-11.

13. The compound III of claim 12, prepared from compound IV by a cross-coupling reaction:

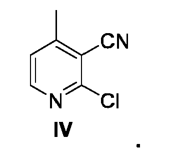

14. According to claim 13, the cross-coupling reaction is an iron-catalyzed Kumada coupling reaction, using compound IV and isopropyl Grignard reagent as the reactants.

15. According to claim 12, the compound III prepared from compound V via a ring closure reaction:

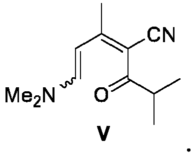

16. According to claim 12, compound III is prepared from compound V via ring closure reaction, and the reaction conditions used are that compound V reacts with an ammonia source followed by an intra-molecular substitution reaction to form compound III.

17. According to claim 16, the ammonia source is comprising of ammonia gas, ammonium acetate, and ammonium chloride.

18. According to claim 15, the compound V prepared from compound VI by a condensation reaction:

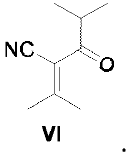

19. According to claim 18, compound V is prepared from compound VI through condensation reaction, using N,N-dimethylformamide dimethyl acetal (DMF-DMA) or N,N- Dimethylformamide dimethyl sulfate adduct (DMF-DMS) as the reactant.

20. According to claim 18, compound VI is prepared from the condensation reaction of compound VII with acetone:
